# EUROPEAN PATENT APPLICATION

(11) **EP 1 782 796 A1**
(43) Date of publication of application: **09.05.2007**
(21) Application number: 05709813.9
(22) Date of filing: 07.02.2005
(51) Int. Cl.: A61K 9/10, A61K 47/02, A61K 47/48

(54) **CARRIER FOR MIGRATION INTO CEREBRAL NEURON CONTAINING METAL COLLOID PARTICLE**

(30) Priority: 06.08.2004 WO PCT/JP2004/011668
(71) Applicant: TISSUE TARGETING JAPAN INC., Nagoya-shi Aichi 4600002 (JP)
(72) Inventor: SAWADA, Makoto, Kasugai-shi, Aichi 4870015 (JP); SUZUKI, Hiromi, Toyota-shi, Aichi 4701201 (JP)
(74) Representative: Grünecker, Kinkeldey, Stockmair & Schwanhäusser Anwaltssozietät
(86) International application number: PCT/JP2005/001761
(87) International publication number: WO 2006/013650

(57) **Abstract**

The present inventors intravenously administered laboratory animals with molecules in which peptides and colloidal metal particles had been bound, to discover that the molecules translocated to the brain, specifically into cerebral neurons. Since activity to translocate into cerebral neurons could not be observed when the metal colloids were not bound, this activity was suggested to be a property of the colloidal metal particles. Desired compounds can be translocated into cerebral neurons by binding them to the colloidal metal particles. Furthermore, by using as carriers conjugate molecules in which peptides having brain-localizing activity are bound to the colloidal metal particles of the present invention, desired compounds can be delivered into cerebral neurons by intravenous administration.

## Description

### Technical Field

The present invention relates to carrier molecules for translocating certain substances into cerebral neurons.

### Background Art

Transport of substances and cells to the brain, which is the center of higher functions, is restricted by a barrier structure called the blood-brain barrier. Therefore the brain was a site where it was difficult to conduct effective treatment, except by surgical operation. Even when white blood cells such as monocytes that circulate through the blood stream are collected and transplanted to adult animals, it is known that the cells do not translocate to the cerebral parenchyma, except when the blood-brain barrier is broken due to external factors (see Non-Patent Document 1).

Achieving an effective concentration of a drug or such by oral administration or injection is more difficult in the brain than in other organs because of the presence of the blood-brain barrier. While an effective drug concentration may be ensured by administering a large dose, this would mean infusing the drug in excess amounts into peripheral blood, which would cause adverse effects such as kidney and liver damage. Therefore, it became necessary to develop a system that selectively transports drugs to the brain. Numerous studies are being carried out in this respect. Most of such research and development involve efforts to enhance brain localization through chemical modification of the drug itself by utilizing a property of cerebrovascular endothelial cells - the higher the lipid solubility of a substance, the more easily it passes through the blood-brain barrier. However, such a method improves drug localization in the brain by several folds at best, which is on the whole, no more than an error range. In the brain, contrary to peripheral organs where substances permeate through the intercellular spaces of vascular endothelial cells, the intercellular spaces of cerebrovascular endothelial cells form special structures called tight junctions and hardly allow permeation of blood components through them. Therefore, transport of substances to the brain must be carried out by permeation after chemical modification of the substances to make them lipid-soluble and directly integratable into the cell membrane. More specifically, this method makes substances permeate directly into cells as there is no alternate route for substance transport to the brain, different from peripheral organs. However, since this mechanism is different from the usual, the efficiency is several thousands to tens of thousands times lower. Therefore, this method cannot be referred to as brain-specific drug transport.

A conceivable method for brain specific transport of pharmaceutical agents is the development of carrier molecules having the property of selectively translocating to brain tissues, and then to administer the pharmaceutical agents in a form bound to such molecules. Known are techniques of using a bioactive peptide receptor as a target of the carrier molecule, or using a monoclonal antibody against a protein, or a substance (ligand) that selectively binds to the protein, as a carrier molecule recognizing the target protein.

However, carrier molecules that translocate specifically to cerebral neurons were not known to be present until now.

[Non-Patent Document 1] Imai, F., Sawada, M., Suzuki, H. et al. Neurosci. Lett. 237 1 pp.49-52 (1997).

### Disclosure of the Invention

### [Problems to be Solved by the Invention]

The present invention was achieved under the above circumstances. An objective of the present invention is to provide carrier molecules that can translocate into cerebral neurons.

### [Means to Solve the Problems]

The present inventors identified molecules having the ability to pass through the blood-brain barrier, and succeeded in identifying peptides having brain-localizing activity. The present inventors examined whether these peptides can be used as carriers that can deliver desired molecules (compounds) to the brain. As a result of intravenously administering molecules in which these peptides are bound to colloidal metal particles to laboratory animals, the present inventors discovered that these molecules translocate to the brain, and furthermore, that they specifically translocate into cerebral neurons. Since the activity of translocating into neurons is not observed when metal colloids are not bound, this activity was suggested to be a property of the colloidal metal particles. More specifically, the present inventors were the first to discover that colloidal metal particles and their complexes have the activity to translocate from the cerebral parenchyma to cerebral neurons. By binding desired compounds to colloidal metal particles, the compounds can be translocated to the cerebral parenchyma.

Additionally, the present inventors discovered that the colloidal metal particles of the present invention are taken up by neurons, but not by microglial cells. Therefore, the colloidal metal particles of the present invention were found to be taken up specifically by neurons. The colloidal metal particles of the present invention can be selective delivery molecules targeting neurons.

Conjugate molecules in which peptides having brain-localizing activity discovered by the present inventors are bound to the colloidal metal particles of the present invention, can be used as carriers to deliver desired molecules into cerebral neurons, for example, by intravenous injection.

As described above, the present inventors found a novel use of colloidal metal particles as carriers for translocation into cerebral neurons, and thereby completed the present invention.

The present invention relates to carrier molecules for translocation into cerebral neurons, and more specifically relates to:
[1] a colloidal metal particle having activity to translocate into a cerebral neuron;
[2] a carrier molecule for translocation into a cerebral neuron, wherein the molecule comprises a colloidal metal particle;
[3] the carrier molecule of [2] having the activity to translocate from cerebral parenchyma into a cerebral neuron;
[4] the carrier molecule of [2] or [3], wherein an avidin molecule is bound to the surface of the colloidal metal particle;
[5] the carrier molecule of any one of [2] to [4], wherein the metal is gold;
[6] a colloidal solution for translocation into a cerebral neuron, in which the carrier molecule of any one of [2] to [5] is present in a dispersed state in solution;
[7] a therapeutic agent for a brain disease, wherein the agent comprises as an active ingredient a carrier molecule of any one of [2] to [5] that supports a therapeutic compound for a brain disease;
[8] a therapeutic agent for a brain disease, wherein the agent comprises as an active ingredient the colloidal solution of [6], in which a carrier molecule supporting a therapeutic compound for a brain disease is present; and
[9] a method for producing a pharmaceutical agent that translocates into a cerebral neuron, wherein the method comprises the step of contacting a therapeutic compound for a brain disease with a carrier molecule of any one of [2] to [5].

More preferably, the above-mentioned carrier molecules have the characteristic of translocating specifically to neurons, and not to microglial cells.

### Brief Description of the Drawings

Fig. 1 shows a schematic structure of a brain-localizing peptide conjugate of the present invention.

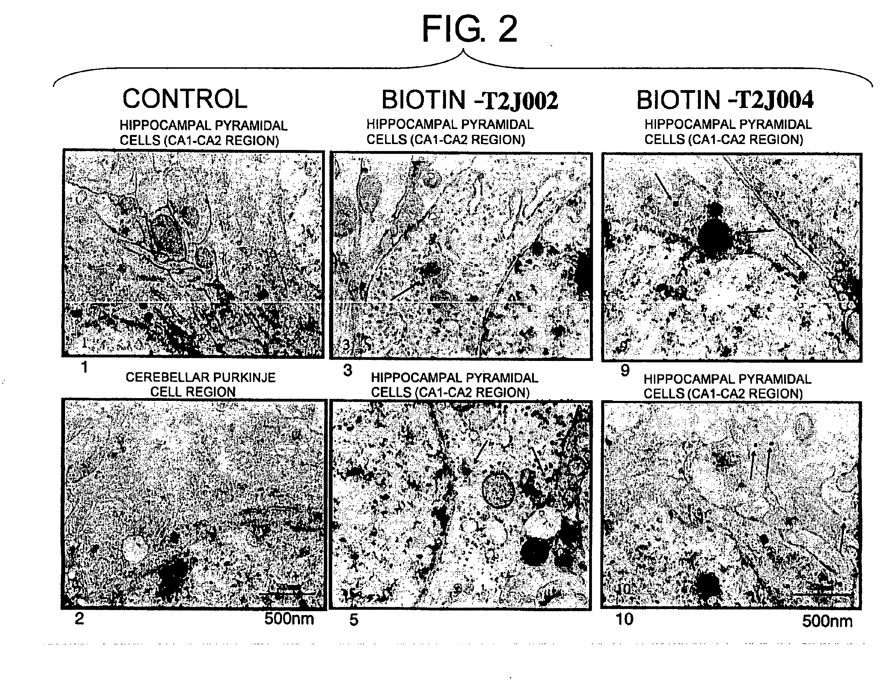

Fig. 2 is a set of electron micrographs showing the presence of conjugates between a peptide and colloidal gold of the present invention in mouse brains. Left (top and bottom): control group (avidin-colloidal gold particles), the label of the colloidal gold particles is not observed in neurons. (Top): hippocampal pyramidal cells (CA1 to CA2 region); (Bottom): cerebellar Purkinje cell region; Center (top and bottom): administration of biotin-conjugated T2J002 + avidin (Av)-colloidal gold particles (gold). The presence of colloidal gold particles in cerebral neurons was seen. Right (top and bottom): administration of biotin-conjugated T2J004 + avidin (Av)-gold particles (gold). Peptide conjugates allowed gold colloids to be transported into the cerebral parenchyma. Colloidal gold particles were shown to be present in cerebral neurons.
Fig. 3 is a set of electron micrographs showing the presence of conjugates between a peptide and gold colloids of the present invention in the pyramidal cell layers CA1-CA2.
   Left: control group (administration of avidin-colloidal gold particles alone); no reaction was observed in the vascular endothelial cells. Center: Biotin + peptide group (1); administration of biotin-conjugated T2J002 + Av-gold. Right: Biotin + peptide group (2); an EM image of the immunoreaction by the pre-embedding method (where biotin-conjugated T2J002 is administered, ultrathin sections are prepared, and then detection is performed with Av-gold for observation). This demonstrated that colloidal gold particles are present in cerebral neurons. No reaction was observed in vascular endothelial cells in the control group (left), whereas, in the experimental groups, deposition of DAB indicating an immunoreaction was observed in endothelial cells (arrows). This reaction was also observed outside the blood vessels (cerebral parenchyma). In group (2) (in which metal colloids were not administered), biotin molecules that pass through the cerebral vascular endothelial cells and biotin molecules that are present in the cerebral parenchyma can be observed due to the action of the brain-targeting peptide, but such molecules cannot be detected in neurons.

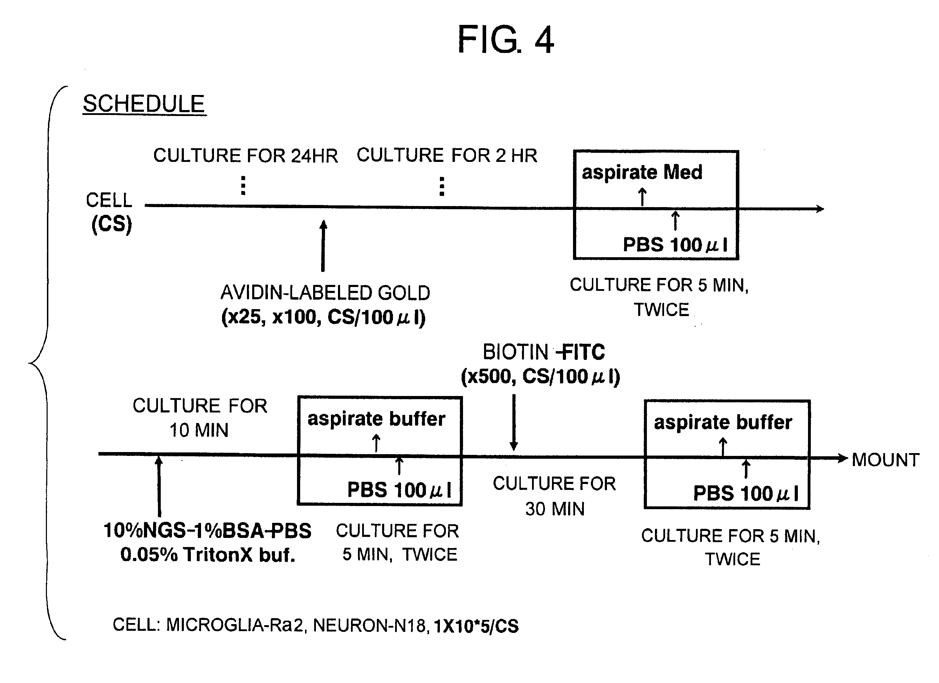

Fig. 4 shows the working schedule for an experiment examining neuron-specific uptake of colloidal gold particles.

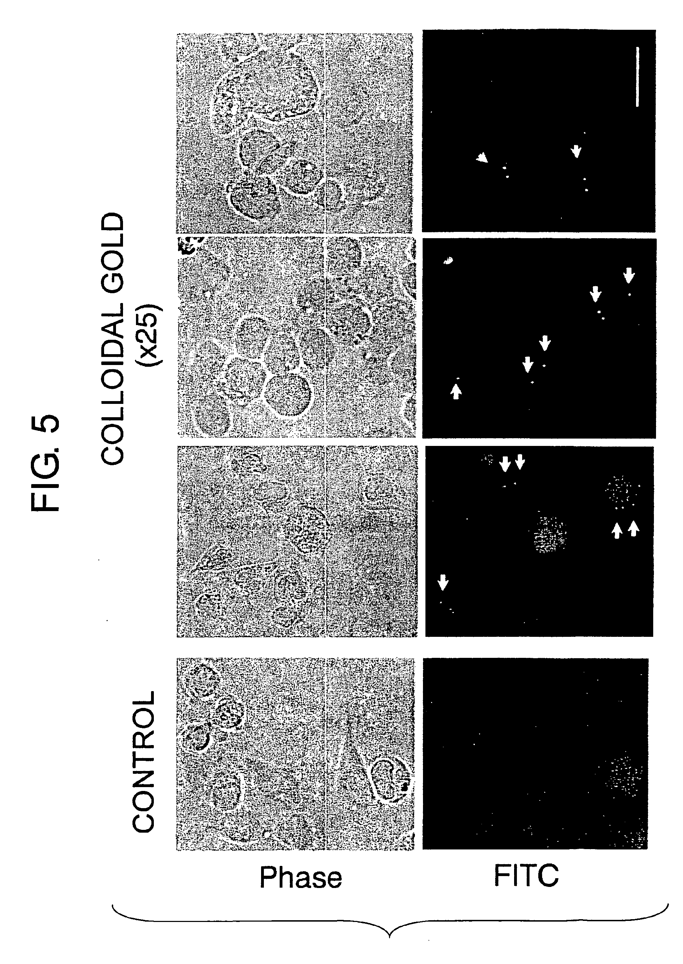

Fig. 5 is a set of photographs showing the uptake of colloidal gold particles by neurons (x 25). In the FITC images in the bottom row, the sites where uptake of colloidal gold particles is observed are indicated by arrows. The scale bar in the figure corresponds to 20 µm.

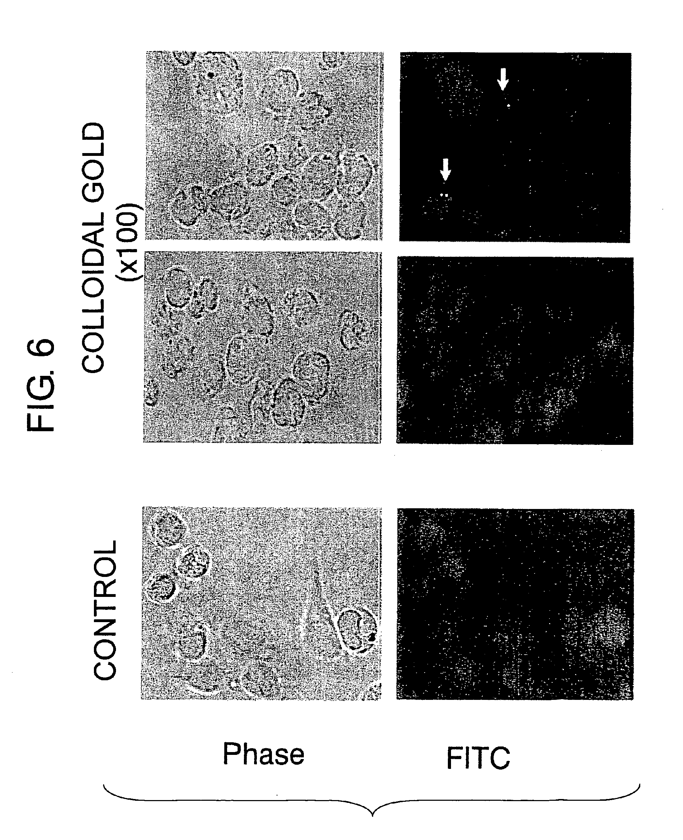

Fig. 6 is a set of photographs showing the uptake of colloidal gold particles by neurons (x 100). In the FITC images in the bottom row, the sites where uptake of colloidal gold particles is observed are indicated by arrows. The scale bar in the figure corresponds to 20 µm.

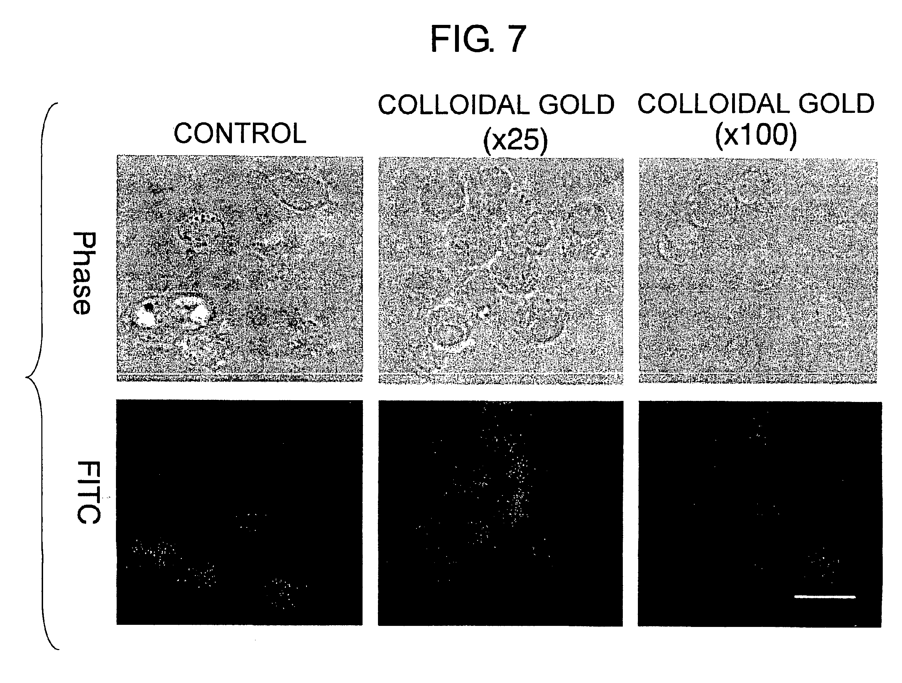

Fig. 7 is a set of photographs showing the uptake of colloidal gold particles by microglial cells (x 25, x 100). The photographs show that uptake of the colloidal gold particles does not occur. The scale bar in the figure corresponds to 20 µm.

### Best Mode for Carrying Out the Invention

The present inventors discovered that colloidal metal particles have the activity to translocate into cerebral parenchyma and particularly into cerebral neurons. Therefore, the present invention provides carrier molecules for translocation into cerebral neurons, which comprise colloidal metal particles. The carriers of the present invention can also be referred to as carriers, transporters, vectors, delivery molecules, or such.

For example, when a carrier molecule of the present invention is administered to a cerebral ventricle, it has the function of translocating into cerebral parenchyma, and particularly into cerebral neurons.

Furthermore, in a preferred embodiment, the carrier molecules of the present invention do not translocate to glial cells. That is, in a preferred embodiment of the carrier molecules of the present invention, such molecules have the activity to specifically translocate into cerebral neurons.

In the present invention, "colloidal metal particles" refer to metal microparticles that can be present in colloidal forms in solution.

Known examples of metals that can be present in colloidal forms in solution include gold (Au), silver (Ag), platinum (Pt), palladium (Pd), copper (Cu), nickel (Ni), rhodium (Rh), iridium (Ir), ruthenium (Ru), cobalt (Co), and osmium (Os).

Examples of metal microparticles of the present invention include microparticles comprising an assembly of metal atoms such as those mentioned above.

Colloidal metal particles of the present invention are not necessarily limited to microparticles comprising a single atom such as those described above, and examples include metal salts, metal hydroxides, and metal oxides (hereinafter referred to as "metal salts" and such). Such examples include salts such as chlorides, sulfates, and nitrates; hydroxides; and oxides of metals such as alkaline earth metals including Mg and Sr, and transition metals including Ag, Au, Fe, Zn, Ti, Cr, Mn, Cu, Ni, Co, and Bi. Among them, salts, hydroxides, or oxides of Fe, Zn, Ti, Al, Sn, Cu, Ni, Si, Mg, Ba, Sr, V, Mn, Mo, Ag, Nb, Zr, Sb, In, or lanthanoids are particularly preferred. These metal salts and such can be used individually or in combinations of two or three to make the colloidal metal microparticles of the present invention.

Examples of the colloidal metal particles of the present invention are preferably colloidal particles composed of gold, cadmium, selenium, iron, or magnetite, and particularly preferred are colloidal gold particles.

The size of the colloidal metal particles of the present invention is not particularly limited, but usually the particle diameter is within the range of 0.5 nm to 100 nm.

For example, when gold is used as the colloidal particles of the present invention, the gold particle size is not particularly limited, but is preferably around 10 nm.

The colloidal metal particles of the present invention may be in a form protected by synthetic polymers and such (polymer-protected colloidal metal particles).

The colloidal metal particles of the present invention can be used as carriers for translocation of desired molecules into cerebral neurons. Usually, by using the carrier molecules of the present invention to support (immobilize) desired compounds, these compounds can be translocated into cerebral neurons.

In the present invention, "to support" refers to bind or to adsorb desired compounds to the surface of colloidal metal particles of the present invention.

Those skilled in the art can use colloidal metal particles to support (immobilize) the desired compounds by applying generally known techniques.

Using naturally occurring polymers such as gelatin, albumin, gum arabic, protarbic acid, and lysalbic acid; and synthetic polymers as protective colloids, metal colloids can be chemically bound and immobilized onto various other substances (compounds). Desired compounds to be translocated into cerebral neurons can be bound (adsorbed) to such polymer-protected metal colloids.

Immobilization of amino group-containing compounds to polymer-protected metal colloids can be carried out, for example, by contacting the polymer-protected metal colloids of the present invention with amino group-containing compounds, reacting the amino groups in the amino group-containing compounds with the polymers adsorbed to the colloidal particles, and then forming chemical bonds between the amino group-containing compounds and the polymers.

Polymer-protected metal colloids can be contacted in a solvent with amino group-containing compounds by using, for example, a means such as stirring. The contact time is generally 10 minutes to 30 hours, and is preferably 30 minutes to 10 hours.

The amino group-containing compound may be any compound so long as it comprises an amino group, and can be selected depending on the use of the substance immobilized on the polymer-protected metal colloids. Examples include homopolymers or copolymers of vinyl compounds comprising primary and/or secondary amino groups; nucleic acids; or proteins. Representative examples of the homopolymers or copolymers include aminostyrene polymers such as poly(4-aminostyrene), and copolymers of 4-aminostyrene and styrene; and crosslinked products thereof, such as ion exchange resins comprising primary and/or secondary amino groups, polyallylamine and crosslinked products thereof, and (C1-C6) aminoalkylated polyacrylamide. Examples of amino group-containing compounds further include amino group-containing nucleic acids such as DNAs and RNAs; nucleic acids such as thymidylic acid to which an amino group has been introduced; isomerase, peroxidase, urease, ATPase, hormones such as insulin, antigens and antibodies; sugars such as heparin, mucopolysaccharide, and keratan sulfate, to which an amino group has been introduced; and lipids to which an amino group has been introduced. When a compound does not comprise an amino group, an amino group can be chemically introduced to this compound so that it can be used as an amino group-containing compound. The amount of an amino group-containing compound to be used is usually 0.1 to 100 moles or preferably 1 to 10 moles with respect to one mole of the hydrazide groups, ester groups, thioester groups, and amide groups in a polymer - the protective colloid in a polymer-protected metal colloid.

Alternatively, when using carrier molecules of the present invention to support a desired compound, the compound does not necessarily have to be directly bound (adsorbed) to the surface of the colloidal metal particles of the present invention. For example, the compound can be bound (adsorbed) to the colloidal metal particles of the present invention by binding avidin (or biotin) to the colloidal metal particles of the present invention, then binding biotin (or avidin) to a desired compound, and then using the interaction between the avidin and biotin.

In a preferred embodiment of the present invention, compounds in which polypeptides having brain-localizing activity, such as the following [1] to [11], have been further bound to the above-mentioned carrier molecules of the present invention can be preferably used. The carrier molecules of the present invention to which the following polypeptides have been bound have the activity to pass through the blood-brain barrier. Therefore, when they are administered to a site other than the brain (for example, by intravenous route) for example, they can translocate to the brain via the blood-brain barrier, and then translocate into cerebral neurons. Accordingly, carrier molecules of the present invention to which the following polypeptides have been bound are very useful as delivery molecules for translocating desired compounds into cerebral neurons. Examples of a preferred embodiment of such molecules include conjugate molecules formed by binding molecules in which biotin has been bound to the following polypeptides and the above-mentioned avidin-bound colloidal metal particles using the interaction between the biotin and avidin.
[1] a polypeptide (brain-localizing polypeptide) having brain-localizing activity, wherein 10% or more of the polypeptide is comprised of basic amino acid residues (K or R);
[2] a polypeptide having brain-localizing activity, wherein the polypeptide comprises a cyclic peptide region, and wherein 10% or more of the cyclic peptide region is comprised of basic amino acid residues (K or R);
[3] a polypeptide having brain-localizing activity, wherein the polypeptide comprises a cyclic peptide region and at least one or more basic amino acid residues (K or R) in the cyclic peptide region;
[4] a polypeptide having brain-localizing activity, wherein the polypeptide comprises a cyclic peptide region and at least one or more basic amino acid residues (K or R), and wherein 80% or more of the remaining amino acid residues in the cyclic region are selected from the following group of amino acid residues: G, A, V, L, S, T, P, Q, H, and N;
[5] a polypeptide having brain-localizing activity, wherein the polypeptide comprises the amino acid motif sequence of:
   X₁-(R or K)-X₃-X₄ or
   X₄-X₃-(R or K)-X₁,
   wherein X₁ denotes S, T, N, P, V, or L; X₃ denotes an arbitrary amino acid; and X₄ denotes G, S, T, C, N, L, Q, or Y;
[6] the polypeptide of any one of [2] to [4], wherein the cyclic peptide region comprises the amino acid motif sequence of [5];
[7] the polypeptide of [5] or [6], wherein the amino acid motif sequence is:
   X₁-(R or K)-X₃-X₄ or
   X₄-X₃-(R or K)-X₁,
   wherein X₁ denotes S, T, N, P, or V; X₃ denotes an arbitrary amino acid; and X₄ denotes an uncharged polar amino acid (G, S, T, C, N, Q, or Y);
[8] the polypeptide of [5] or [6], wherein the amino acid motif sequence is:
   X₁-(R or K)-X₃-X₄ or
   X₄-X₃-(R or K)-X₁,
   wherein X₁ denotes S, T, P, or L; X₃ denotes an arbitrary amino acid; and X₄ denotes S, T, C, L, or Q;
[9] the polypeptide of any one of [1] to [8], wherein the polypeptide has transmigration-inducing activity;
[10] the polypeptide of any one of [1] to [8], wherein the polypeptide has activity to bind to a cerebrovascular endothelial cell;
[11] a polypeptide of any one of (a) to (c) described below:
   (a) a polypeptide comprising the amino acid sequence of any one of SEQ ID NOs: 1 to 12,
   (b) a polypeptide comprising a peptide region cyclized by a disulfide bond formed between cysteine residues on both ends of the polypeptide of (a), and
   (c) a polypeptide having brain-localizing activity, and comprising an amino acid sequence with one or several amino acid additions, deletions, or substitutions in the amino acid sequence of any one of SEQ ID NOs: 1 to 12.

The colloidal metal particles of the present invention are usually present in a dispersed state in solution (colloidal form). Colloidal metal particles of the present invention which are present in such a colloidal form (colloidal solution) may also be comprised in the present invention. Specifically, the present invention provides colloidal solutions for translocation into cerebral neurons, in which the carrier molecules of the present invention are present in a dispersed state in solution.

Examples of the colloidal solutions of the present invention include solutions produced by mixing the colloidal metal particles of the present invention with aqueous solvents or organic solvents (such as alcohol or ethanol). By considering the type of metal particle and such, those skilled in the art would appropriately know solvent compositions, conditions, and such that enable metal microparticles to exist as colloids.

Furthermore, by using the carrier molecules of the present invention, desired pharmaceutical agents can be translocated into the cerebral neurons. For example, by using the carrier to support a compound (pharmaceutical composition) that has a therapeutic effect on a brain disease, the compound can be delivered efficiently into the cerebral neurons, and made to exert effective therapeutic effects. Carriers supporting a compound (pharmaceutical composition) are expected to be therapeutic agents for brain diseases.

Accordingly, the present invention provides therapeutic agents for brain diseases, which comprise as an active ingredient a drug (such as a therapeutic compound for a brain disease) supported on a carrier molecule for translocation into cerebral neurons. Herein, "supported" may refer to conditions in which a drug is directly bound to (adsorbed to) a carrier molecule, conditions in which a drug (pharmaceutical composition) is contained within a carrier molecule, or conditions in which a drug is mixed with a carrier molecule. More specifically, a desired compound may be contained within a liposome, microcapsule, or such, and the liposome or microcapsule may be bound to the carrier molecule of the present invention.

The present invention also provides methods for producing pharmaceutical agents that translocate into cerebral neurons, which methods comprise the step of contacting carrier molecules of the present invention for translocation into cerebral neurons or colloidal solutions of the present invention with therapeutic compounds for brain diseases. Usually, "contacting" is suitably carried out according to the conditions of the carrier molecules of the present invention. For example if the carrier molecules of the present invention are in a purified state, contacting can be performed by adding therapeutic compounds for brain diseases to the purified preparations. Contacting can also be carried out by adding therapeutic compounds for brain diseases to the colloidal solutions of the present invention. Therapeutic compounds can be bound to (adsorbed to) the carrier molecules of the present invention by these methods. Methods for producing pharmaceutical agents that translocate into cerebral neurons comprising various types of steps in the above-mentioned methods are also a preferred embodiment of the production methods of the present invention.

The pharmaceutical agents of the present invention may comprise carrier molecules of the present invention or colloidal solutions in which the carrier molecules are present as active ingredients, or metal microparticles of the present invention may be formulated using a known pharmaceutical production method. For example, the microparticles can be formulated into pharmaceutical formulations suitable for effective administration into the body, such as injections, transnasal formulations, transdermal formulations, and oral agents, but preferably injections, by suitably combining with appropriate conventionally used carriers or vehicles such as sterilized water, physiological saline, vegetable oil (for example, sesame oil and olive oil), coloring agent, emulsifier (for example, cholesterol), suspending agent (for example, gum arabic), surfactant (for example, polyoxyethylene hardened castor oil surfactants), solubilizing agent (for example, sodium phosphate), stabilizer (for example, sugars, sugar alcohols, and albumin), or preservative (for example, paraben). For example, injection formulations can be provided as freeze-dried products, solutions for injections, or such.

Furthermore, administration into the body can be performed, for example, by intraarterial injection, intravenous injection, or subcutaneous injection, and also intranasally, transbronchially, intramuscularly, or orally using methods known to those skilled in the art. Direct administration to a diseased site, such as the cerebral parenchyma, is also possible.

A physician can appropriately determine the dose of a pharmaceutical agent or a pharmaceutical composition of the present invention after considering the type of dosage form, administration method, the age, weight, and symptoms of the patient, and such.

The present invention also relates to methods for treating brain diseases, which comprise the step of administering to patients the carrier molecules for translocation into cerebral neurons that support therapeutic compounds for brain diseases. Alternatively, the present invention relates to methods for treating brain diseases, which comprise the step of administering to patients colloidal solutions in which carrier molecules supporting therapeutic compounds for brain diseases are present.

Furthermore, administration into the body can be performed, for example by intraarterial injection, intravenous injection, or subcutaneous injection, and also intranasally, transbronchially, intramuscularly, or orally using methods known to those skilled in the art. However, local administration (for example, administration to the cerebral ventricle) is preferred. When carrier molecules of the present invention which have been bound to polypeptides having brain-localizing activity are used, they will be effective even when administered intraarterially or intravenously, for example. The dose may vary depending on the age, weight, and symptoms of the patient, administration method, and such, but those skilled in the art can appropriately select a suitable dose.

Carrier molecules of the present invention can be applied to the following cases as a therapeutic strategy for treating cranial nerve diseases.

### 1) Replacement therapies that supplement enzymes and bioactive proteins whose amounts and activities have decreased due to deletions or mutations

These therapies are performed for various brain diseases caused by deficiencies of particular enzymes or proteins in the brain (particularly, in neurons) due to genetic deletions or mutations, by injecting carrier molecules supporting the deficient proteins and enzymes. For degenerative loss of particular neurons in Parkinson's disease and Alzheimer's disease, genes that promote synthesis of neurotransmitters which become deficient due to the degenerative loss of nerves may be used; for example, genes of enzymes involved in dopamine biosynthesis including tyrosine hydroxylase and biopterin synthase may be used for Parkinson's disease.

### 2) Protective therapies for protecting neurons that would otherwise be lost by degeneration or such and for strengthening their functions

These therapies are performed by injecting cells expressing genes of neurotrophic factors, such as NGF, BDNF, GDNF, and NT3 (recent findings show that BDNF and GNNF are particularly effective for Parkinson's disease), which suppress neuron death by various causes including degenerative diseases and cerebral ischemia, and promote the regeneration of neurites. Furthermore, therapies for diseases that involve immune cells, such as multiple sclerosis, are performed by introducing carrier molecules that support the TGF-β gene or IL-10 gene, which have immunosuppressive effects.

### 3) Methods for removing tumors, blood clots and such

These methods are performed by expressing factors that have antitumor effects, or by transferring cells that carry an antitumor agent into the brain. For removal of blood clots, carrier molecules supporting fibrinolytic enzymes can be introduced.

### 4) Methods for introducing effective drugs exclusively into the brain

Among drugs that act on the nervous system, some have high peripheral toxicity, some act on the peripheral nervous system, and others cannot easily pass through the blood-brain barrier; therefore, drug delivery systems that are specific for the brain are necessary. Using the carrier molecules of the present invention, drugs may be administered specifically to the brain, with little effect on peripheral organs.

Furthermore, the present invention relates to uses of carrier molecules for translocation into cerebral neurons that support therapeutic compounds for brain diseases, in the production of therapeutic agents for brain diseases. Alternatively, the present invention relates to uses of colloidal solutions in which carrier molecules supporting therapeutic compounds for brain diseases are present, in the production of therapeutic agents for brain diseases.

All prior art references cited herein are incorporated herein by reference.

### Examples

Herein below, the present invention will be specifically described with reference to Examples, but it is not to be construed as being limited thereto.

### [Example 1] Brain-localizing peptide conjugates

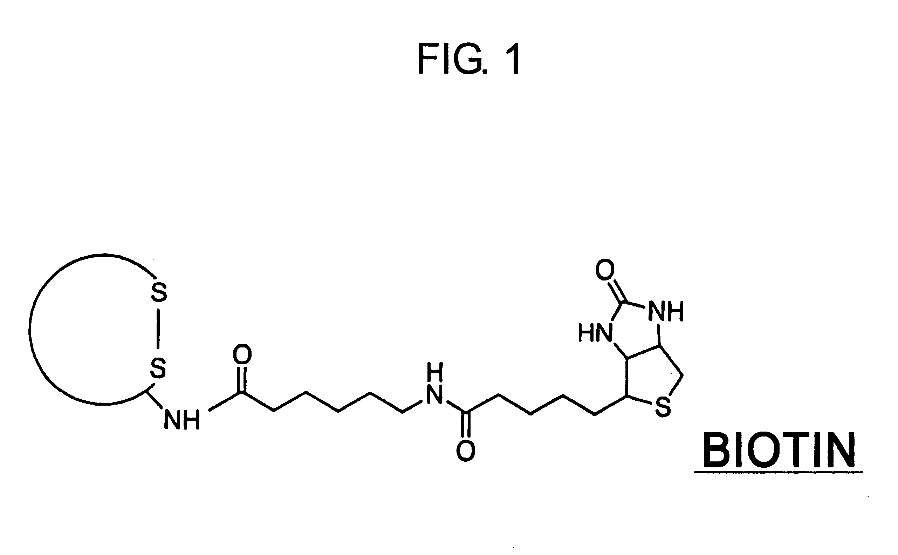

The present inventors produced brain-localizing peptide conjugates. The conjugates are biotinylated molecules that can have a cyclic structure due to the disulfide bond formation between cysteine residues in a polypeptide molecule having the above-mentioned brain-localizing activity. A more specific example is the structure shown in Fig. 1. The conjugates have affinity for avidin compounds.

The conjugates were bound to colloidal gold particles and administered to mice, and the mice were subjected to experiments that evaluate the brain-localizing activity of the peptides of the present invention (observation of brain tissue sections under a transmission electron microscope). The protocol for preparing transmission electron microscopy samples is shown in Table 1.

**Table 1**

| [Block preparation] | |
|---|---|
| (i) prefixation: | 2.5% glutaraldehyde solution in 0.1 M PB at 4°C |
| (ii) washing: | 0.1 M PBS, cooled once or twice on ice |
| (iii) postfixation: | 1% osmium tetroxide solution in 0.1 M PB, cooled on ice for approximately 1 hour |
| (iv) dehydration: | ethanol series 70 → 80 → 90 → 95 → 100 → 100 → 100 (%), 10 minutes each |
| (v) resin embedding: | Epok 812 resin polymerization at 35°C for 12 hours; 45°C, 12 hours; and 60°C, 20 hours |

| [Section preparation] | |
|---|---|
| (i) | trimming |
| (ii) | preparation of thick sections (2 to 3 µm thick) using a glass knife |
| (iii) | preparation of ultra-thin sections (50 to 100 nm thick) using a diamond knife |
| (iv) | electron staining: 2% uranium acetate and then 1% lead citrate (3 minutes each) |
| (v) | transmission electron microscope observation (acceleration voltage of 80 kV) |

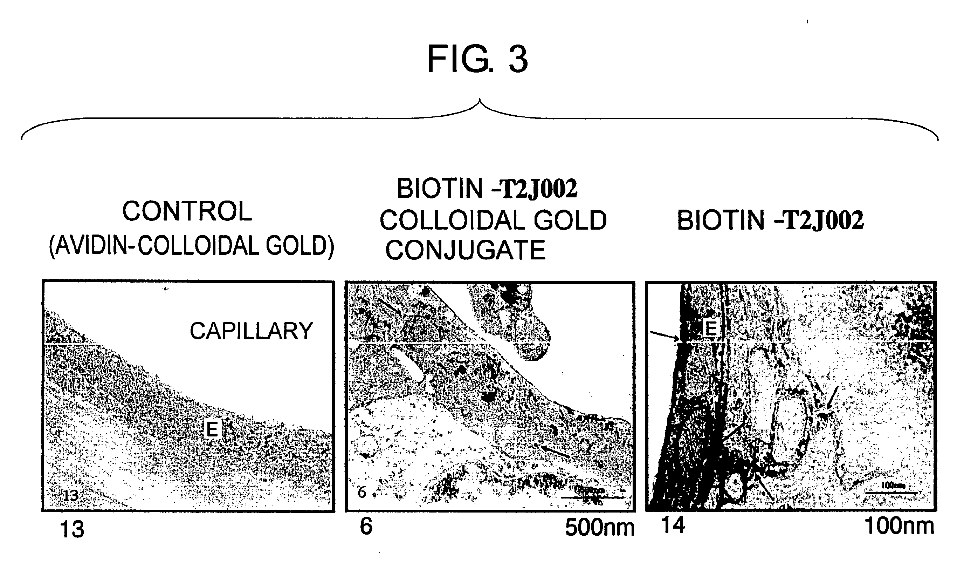

In the present experiment, T2J002 (CSLNTRSQC/SEQ ID NO: 2) and T2J004 (CVVRHLQQC/SEQ ID NO: 4) were used as peptides having brain-localizing activity. The electron micrographs of mouse brain tissues (cells) are shown in Figs. 2 and 3. The arrows indicate positions of the gold particle-bound peptide molecules of the present invention. The results showed that the peptide conjugates of the present invention successfully transported the colloidal gold particles to the cerebral parenchyma. The colloidal gold particles were found to translocate into cerebral neurons.

### [Example 2] Neuron-specific uptake of colloidal gold particles

Avidin-labeled colloidal gold particles (x 25, x 100, CS/100 µL) were added to a variety of cells (microglial-Ra2 cells, neuron-N18, and 1 x 10⁵/CS cells), the cells were cultured, and after adding FITC-labeled biotin, the cells were further cultured. Specifically, the experiment was carried out according to the schedule shown in Fig. 4.

Following the procedure shown in Fig. 4, uptake of colloidal gold particles of the present invention into the neurons was observed under a fluorescent microscope.

The results confirmed that the colloidal gold particles were taken up specifically by neurons (Figs. 5 and 6). On the other hand, uptake of colloidal gold particles by microglial cells was not observed (Fig. 7).

Since animal experiments in the above Example had confirmed that when colloidal gold particles were injected through the carotid artery, they were taken up by neurons in the brain, the findings matched with those of the present Example.

### Industrial Applicability

If drugs and such can be effectively transported into cerebral neurons by using the carrier molecules of the present invention for translocation into cerebral neurons, drugs that had been difficult to use since they were metabolized or drugs that had been difficult to pass through the blood-brain barrier may become usable. Furthermore, drugs that had to be used at high concentrations may be used at a reduced dose.

Moreover, the present invention is very useful for treating diseases for which drug efficacy needs to be exerted in cerebral neurons.

The present invention leads to the development of pharmaceutical products that can maximize drug efficacy, and to the establishment of methods using such pharmaceuticals. The present invention can also enable the development of novel therapeutic agents for brain diseases such as brain tumors.

## Claims

1. A colloidal metal particle having activity to translocate into a cerebral neuron.

2. A carrier molecule for translocation into a cerebral neuron, wherein the molecule comprises a colloidal metal particle.

3. The carrier molecule of claim 2 having the activity to translocate from cerebral parenchyma into a cerebral neuron.

4. The carrier molecule of claim 2 or 3, wherein an avidin molecule is bound to the surface of the colloidal metal particle.

5. The carrier molecule of any one of claims 2 to 4, wherein the metal is gold.

6. A colloidal solution for translocation into a cerebral neuron, in which the carrier molecule of any one of claims 2 to 5 is present in a dispersed state in solution.

7. A therapeutic agent for a brain disease, wherein the agent comprises as an active ingredient a carrier molecule of any one of claims 2 to 5 that supports a therapeutic compound for a brain disease.

8. A therapeutic agent for a brain disease, wherein the agent comprises as an active ingredient the colloidal solution of claim 6, in which a carrier molecule supporting a therapeutic compound for a brain disease is present.

9. A method for producing a pharmaceutical agent that translocates into a cerebral neuron, wherein the method comprises the step of contacting a therapeutic compound for a brain disease with a carrier molecule of any one of claims 2 to 5.


SEQUENCE LISTING

<110>  TISSUE TARGETING JAPAN INC.

<120>  CARRIER FOR MIGRATION INTO CEREBRAL NEURON CONTAINING METAL COLLOID
PARTICLE

<130>  TTJ-A0402P

<150>  PCT/JP2004/11668
<151>  2004-08-06

<160>  12

<170>  PatentIn version 3.1

<210>  1
<211>  9
<212>  PRT
<213>  Artificial

<220>
<223>  an artificially synthesized sequence

<400>  1
Cys Ser Asn Leu Leu Ser Arg His Cys
1               5

<210>  2
<211>  9
<212>  PRT
<213>  Artificial

<220>
<223>  an artificially synthesized sequence

<400> 2
Cys Ser Leu Asn Thr Arg Ser Gln Cys
1               5

<210> 3
<211> 9
<212> PRT
<213> Artificial

<220>
<223> an artificially synthesized sequence

<400> 3
Cys Val Ala Pro Ser Arg Ala Thr Cys
1               5

<210> 4
<211> 9
<212> PRT
<213> Artificial

<220>
<223> an artificially synthesized sequence

<400> 4
Cys Val Val Arg His Leu Gln Gln Cys
1               5

<210> 5
<211> 9
<212> PRT
<213> Artificial

<220>
<223>  an artificially synthesized sequence

<400>  5
Cys Val Leu Arg His Leu Gln Gln Cys
1               5

<210>  6

<211>  9
<212>  PRT
<213>  Artificial

<220>
<223>  an artificially synthesized sequence

<400>  6
Cys Arg Gln Leu Val Gln Val His Cys
1               5

<210>  7
<211>  9
<212>  PRT
<213>  Artificial

<220>
<223>  an artificially synthesized sequence

<400>  7
Cys Gly Pro Leu Lys Thr Ser Ala Cys
1               5

<210> 8
<211> 9
<212> PRT
<213> Artificial

<220>
<223> an artificially synthesized sequence

<400> 8
Cys Leu Lys Pro Gly Pro Lys His Cys
1               5

<210> 9
<211> 9
<212> PRT
<213> Artificial

<220>
<223> an artificially synthesized sequence

<400> 9
Cys Arg Ser Pro Gln Pro Ala Val Cys
1               5

<210> 10
<211> 9
<212> PRT
<213> Artificial

<220>
<223> an artificially synthesized sequence

<400> 10
Cys Asn Pro Leu Ser Pro Arg Ser Cys

1                   5

<210>  11
<211>  9
<212>  PRT
<213>  Artificial

<220>
<223>  an artificially synthesized sequence

<400>  11
Cys Pro Ala Gly Ala Val Lys Ser Cys
1                   5

<210>  12
<211>  9
<212>  PRT
<213>  Artificial

<220>
<223>  an artificially synthesized sequence

<400>  12
Cys Pro Ala Gly Ala Leu Lys Ser Cys
1                   5